# EUROPÄISCHE PATENTANMELDUNG

(11) **EP 4 427 796 A1**
(43) Veröffentlichungstag der Anmeldung: **11.09.2024**
(21) Anmeldenummer: 23160611.2
(22) Anmeldetag: 07.03.2023
(51) Int. Cl.: A61M 60/562, A61M 60/403, A61M 60/508

(54) **SYSTEM MIT EINER BLUTPUMPE UND EINER STEUEREINHEIT SOWIE VERFAHREN ZUM BETRIEB EINER BLUTPUMPE**

(71) Anmelder: Berlin Heart GmbH, 12247 Berlin (DE)
(72) Erfinder: PALAMARCHUK, Evgenii, 12247 Berlin (DE); STRAUCH, Carsten, 12247 Berlin (DE)
(74) Vertreter: Pfenning, Meinig & Partner mbB

(57) **Zusammenfassung**

Die Erfindung bezieht sich auf ein System umfassend eine Blutpumpe (1) mit einem um eine Rotationsachse (8) rotierend antreibbaren Rotor (3) zur Blutförderung sowie eine Steuereinheit (2) für eine Blutpumpe, wobei die Steuereinheit dazu eingerichtet ist, den Rotor zeitlich aufeinanderfolgend, insbesondere mehrfach abwechselnd, in einem ersten Fördermodus (A) sowie wenigstens in einem zweiten Fördermodus (2) zu betreiben.

Die Aufgabe, Totwassergebiete im Bereich des Rotors und eventuelle Thrombenbildung zu vermeiden, wird gemäß der Erfindung dadurch gelöst, dass in dem zweiten Fördermodus (B) die Rotationsrichtung (4, 5) des Rotors gegenüber dem Betrieb im ersten Fördermodus (A) umgekehrt ist.

## Beschreibung

Die Erfindung liegt auf dem Gebiet der Medizintechnik und des Maschinenbaus sowie der Strömungsmechanik und ist mit besonderem Vorteil im Bereich von Blutpumpen anwendbar.

Blutpumpen zur Unterstützung oder zum Ersatz der Herzfunktion eines Patienten werden bereits seit einiger Zeit mit Erfolg eingesetzt. Dabei sind verschiedene Formen von Pumpen bekannt, von denen einige implantierbar sind und andere stationär angeordnet oder am Körper eines Patienten getragen werden. In vielen Fällen ist es sinnvoll, Blutpumpen wenn möglich zu implantieren. Unter diesen implantierbaren Pumpen stellen die VAD (Ventricular Assist Devices)-Pumpen eine besondere Gruppe dar. Diese können derart implantiert werden, dass sie an einen Herzventrikel angeschlossen werden und von dort Blut ansaugen, das in das Gefäßsystem des Patienten geleitet wird.

Solche VAD-Pumpen sind in vielen Fällen als Rotationspumpen ausgebildet und weisen demnach einen antreibbaren Rotor zur Blutförderung auf. Beim Betrieb derartiger Pumpen ist es sinnvoll, Maßnahmen zu ergreifen, um schlecht durchströmte sog. Totwassergebiete innerhalb der Pumpe oder in deren direktem Umfeld zu vermeiden oder zu minimieren. Solche Totwassergebiete können ggf. zur Thrombenbildung führen oder beitragen.

Aus dem Stand der Technik sind Verfahren zum Betrieb von implantierbaren VAD-Rotationspumpen bekannt, bei denen die Drehzahl der Pumpe variiert wird, um einerseits einen pulsatilen Betrieb einer solchen Pumpe zu ermöglichen, der die natürliche Arbeitsweise eines funktionierenden Herzens imitiert oder unterstützt, und andererseits der Thrombenbildung entgegenzuwirken.

In diesem Zusammenhang sind beispielsweise die Patentschriften US 9,433,714 B2, US 9,433,717 B2, US 9,011,312 und EP 2 618 863 B1 bekannt geworden. Diese Schriften offenbaren jeweils Verfahren, bei denen eine Drehzahlsteuerung die Drehzahl einer Blutpumpe in bestimmten Grenzen und nach bestimmten zeitlichen Mustern verringert und wieder erhöht.

Der vorliegenden Erfindung liegt die Aufgabe zugrunde, ein System mit einer Blutpumpe zu schaffen, das die Ausprägung, Häufigkeit und/oder Wahrscheinlichkeit der Bildung von Totwassergebieten gegenüber dem Stand der Technik weiter reduziert.

Die Aufgabe wird mit den Merkmalen der Erfindung gemäß Patentanspruch 1 durch ein System mit einer Blutpumpe und einer Steuereinheit gelöst. Zudem bezieht sich die Erfindung auf ein maschinenlesbares Speichermedium mit Anweisungen für eine Steuereinheit, die einen entsprechend erfindungsgemäßen Betrieb einer Blutpumpe ermöglichen sowie auf ein Verfahren zum Betrieb einer Blutpumpe mit einem antreibbaren Rotor.

Im Einzelnen bezieht sich die Erfindung somit auf ein System, umfassend eine Blutpumpe mit einem um eine Rotationsachse rotierend antreibbaren Rotor zur Blutförderung sowie eine Steuereinheit für eine Blutpumpe, wobei die Steuereinheit dazu eingerichtet ist, den Rotor zeitlich aufeinanderfolgend, insbesondere mehrfach abwechselnd, in einem ersten Fördermodus A sowie wenigstens in einem zweiten Fördermodus B zu betreiben.

Die Aufgabe wird gemäß der Erfindung dadurch gelöst, dass in dem zweiten Fördermodus die Rotationsrichtung des Rotors gegenüber dem Betrieb im ersten Fördermodus umgekehrt ist.

Durch die Umkehrung der Rotationsrichtung des Rotors gegenüber dem Betrieb im ersten Fördermodus wird die Anströmrichtung des Rotors umgekehrt und damit qualitativ gegenüber einer bloßen Drehzahländerung sprunghaft verändert. Es können damit in dem Rotor andere Bereiche verstärkt der Blutströmung ausgesetzt werden, als dies bei einer nur graduellen Änderung der Drehzahl bei gleichbleibender Drehrichtung möglich ist. Dadurch wird die Ausbildung von Totwassergebieten in Bereichen des Rotors verringert, die durch nur eine betragsmäßige Änderung der Drehzahl nicht erreicht werden. Durch die Erfindung werden somit gegenüber dem Stand der Technik zumindest zeitweise Gebiete innerhalb des Rotors zuverlässig gut durchströmt und somit von der Ausbildung von Totwassergebieten freigehalten, in denen sich gemäß dem Stand der Technik Risiken der Thrombenbildung ergeben können.

Eine vorteilhafte Ausgestaltung der Erfindung kann vorsehen, dass der Rotor derart eingerichtet ist, dass die Förderrichtung der Blutpumpe im zweiten Fördermodus B dieselbe ist wie im ersten Fördermodus A.

Da üblicherweise bei radial oder diagonal (gemischt radial/axial) fördernden Rotationspumpen die Förderung des Blutes durch eine Zentrifugalkraft innerhalb des Rotors gewährleistet wird, bleibt die Förderrichtung des Blutes in der Pumpe bei solchen Pumpen unabhängig von der Rotationsrichtung des Rotors unverändert. Es kann sich der Wirkungsgrad der Pumpe in Abhängigkeit von der Rotationsrichtung ändern, jedoch wird eine Rückströmung des Blutes bei der Änderung der Rotationsrichtung verhindert. Es ist somit in vielen Fällen sinnvoll, dass der Rotor zur Förderung einer Flüssigkeit, insbesondere von Blut in radialer oder diagonaler Richtung eingerichtet ist. Die Erfindung kann jedoch in einigen Fällen auch bei axial fördernden Rotationspumpen mit Vorteil eingesetzt werden.

Es kann zudem vorgesehen sein, dass der Rotor ein oder mehrere Förderelemente, insbesondere in Form von Schaufeln, aufweist.

Derartige Förderelemente können beispielsweise an einer Nabe befestigt sein und radial von dieser nach außen ragen oder in einer anderen Ausführungsform können Förderelemente auch auf einem scheibenförmigen Träger mit einer zentralen Durchströmöffnung angeordnet sein. Die Förderelemente weisen üblicherweise Förderflächen auf, die auf einer Druckseite des jeweiligen Förderelementes angeordnet sind, wobei sich jedoch bei einem Drehzahlumkehrbetrieb/zweiten Fördermodus auch auf der gegenüberliegenden Seite der Förderelemente Förderflächen ergeben, die im Normalbetrieb/ersten Fördermodus auf der Saugseite des Rotors angeordnet sind. Förderelemente und gegebenenfalls Förderflächen können gerade/eben oder in einer oder mehreren Richtungen gekrümmt ausgebildet sein, wobei die Förderflächen sich zumindest teilweise in radialer Richtung des Rotors erstrecken.

Somit kann eine Implementierung der Erfindung vorsehen, dass wenigstens eine, insbesondere zwei oder mehr als zwei Schaufeln des Rotors oder die im ersten Fördermodus A auf der Druckseite der jeweiligen Schaufeln liegenden Förderflächen sich jeweils, in Blickrichtung parallel zur Rotationsachse gesehen, von einem ersten Punkt, der einen ersten Abstand zur Rotationsachse aufweist, in geradem oder gekrümmtem Verlauf bis zu einem zweiten Punkt erstrecken, der einen zweiten, größeren Abstand zur Rotationsachse aufweist als der erste Punkt.

In Blickrichtung der Rotationsachse des Rotors gesehen, können eines oder mehrere der Förderelemente beispielsweise die Form eines Abschnitts einer Spirale aufweisen und symmetrisch um eine Durchströmöffnung angeordnet sein, die zentral innerhalb eines Trägers der Schaufeln gebildet ist. Das zu fördernde Blut strömt dann durch die Durchströmöffnung ein und wird im Rahmen der Rotationsbewegung des Rotors durch Zentrifugalkräfte nach außen gefördert. Die einzelnen Förderelemente weisen dann Anströmkanten oder Anströmbereiche auf, die an dem jeweils radial innen liegenden Ende eines jeden Förderelementes angeordnet sind. Die Förderelemente einer Rotationspumpe sind üblicherweise derart gestaltet, dass die Pumpe in einer ersten Rotationsrichtung eine wesentlich höhere Effizienz aufweist als in einer zweiten Rotationsrichtung des Rotors. Die radial innen liegenden Anströmkanten der Förderelemente werden durch die zu fördernde Flüssigkeit in Abhängigkeit von der Drehzahl in bestimmten Grenzen unterschiedlich angeströmt. Durch eine Umkehrung der Rotationsrichtung werden tatsächlich zusätzlich völlig neue Bereiche und gegebenenfalls auch verschiedene Seiten eines Förderelementes einer Strömung ausgesetzt, so dass bei wechselnder Rotationsrichtung der gesamte Bereich in der Umgebung des radial innen liegenden Anströmbereichs jedes Förderelementes zu bestimmten Zeiten einer verstärkten Strömung ausgesetzt wird und damit in größerem Umfang eine dauerhafte Ausbildung von Totwassergebieten verhindert werden kann.

Der Rotor einer implantierbaren Blutpumpe ist oft derart asymmetrisch gestaltet, dass beim Betrieb in einer ersten Rotationsrichtung des Rotors, die beispielsweise in einem ersten Fördermodus angewendet wird, eine wesentlich höhere Fördereffizienz erreicht wird, als beim Betrieb in der entgegengesetzten Rotationsrichtung, die in einem zweiten Fördermodus angewendet werden kann. Hierzu kann beispielsweise vorgesehen sein, dass die Schaufeln des Rotors oder die im ersten Fördermodus auf der Druckseite der jeweiligen Schaufeln liegenden Förderflächen sich jeweils, in Blickrichtung parallel zur Rotationsachse gesehen, von der Rotationsachse aus radial nach außen erstrecken und insbesondere zumindest abschnittsweise in ihrem Verlauf eine bezüglich des Rotors tangentiale Komponente aufweisen.

Der Betrieb einer solchen Blutpumpe wird dann derart gestaltet werden, dass sie überwiegend im ersten Fördermodus mit einer Rotationsrichtung betrieben wird, die der optimierten Effizienz der Pumpe entspricht. Zeitabschnitte, in denen die Pumpe in der entgegengesetzten Rotationsrichtung des Rotors betrieben wird, werden eine geringere Zeitdauer einnehmen und gegebenenfalls regelmäßig oder unregelmäßig kürzere Zeitintervalle betreffen.

Die zeitlichen Perioden, in denen die Pumpe im ersten Fördermodus betrieben wird, können beispielsweise mehr als 80 %, insbesondere mehr als 90 %, weiter insbesondere mehr als 95 %, weiter insbesondere mehr als 99 % der Betriebszeit umfassen, während die zeitlichen Perioden des Betriebs in dem zweiten Fördermodus weniger als 20 %, insbesondere weniger als 10 %, weiter insbesondere weniger als 5 % und weiter insbesondere als 1 % der Betriebszeit umfassen können. Einzelne Zeitabschnitte, in denen die Blutpumpe im zweiten Fördermodus betrieben wird, können beispielsweise kürzer als eine Sekunde, insbesondere kürzer als 0,5 Sekunden, weiter insbesondere kürzer als 0,2 oder kürzer als 0,1 Sekunde sein. Die Häufigkeit, mit der im Betrieb Zeitabschnitte des zweiten Fördermodus eingesteuert werden, kann beispielsweise so gestaltet sein, dass wenigstens alle zwei Sekunden, insbesondere wenigstens alle 1,5 Sekunden, ein Zeitabschnitt des zweiten Fördermodus stattfindet. Ein regelmäßiger zeitlicher Abstand zwischen zwei Zeitabschnitten des zweiten Fördermodus kann somit beispielsweise kleiner als 5 Sekunden, insbesondere kleiner als 3 Sekunden sein. Es kann in einer anderen Ausgestaltung jedoch auch sein, dass die Zeitabschnitte des zweiten Fördermodus seltener eingesteuert werden und beispielsweise seltener als einmal pro Minute, insbesondere auch seltener als einmal in 10 Minuten oder seltener als 1mal pro Stunde stattfinden. Typische Drehzahlen im ersten Fördermodus können beispielsweise zwischen 2000rpm und 5300rpm liegen, während Drehzahlen im zweiten Fördermodus beispielsweise betragsmäßig über 1000 rpm bis 2500 oder bis 4000rpm liegen können. Die Drehzahlen im Umkehrmodus/zweiten Betriebsmodus können beispielsweise auch betragsmäßig unter 500rpm oder sogar unter 200rpm liegen.

Die Häufigkeit, mit der das Einsteuern von Zeitabschnitten des zweiten Fördermodus notwendig ist, hängt von der geometrischen Gestaltung des Rotors bzw. der Förderelemente ab. Es kann in einer besonderen Ausgestaltung des Betriebs auch vorgesehen sein, dass im ersten Fördermodus kurz vor Einleiten des zweiten Fördermodus die Drehzahl angehoben wird, um danach bis auf null abgesenkt und auf negative Drehzahlen, d. h., auf einen Drehzahlumkehrbetrieb gebracht zu werden. Nach Beendigung des zweiten Fördermodus, der beispielsweise 0,1 oder 0,5 Sekunden oder weniger als 1 Sekunde dauern kann, wird dann die Drehzahl wieder umgekehrt und auf eine konstante Drehzahl des ersten Fördermodus angehoben.

Die Erfindung bezieht sich außer auf ein System der oben beschriebenen Art auch auf eine Steuereinheit für eine Blutpumpe mit einem um eine Rotationsachse rotierend antreibbaren Rotor zur Blutförderung, die dazu eingerichtet ist, den Rotor zeitlich aufeinanderfolgend in einem ersten Fördermodus sowie wenigstens in einem zweiten Fördermodus zu betreiben, wobei in dem zweiten Fördermodus die Rotationsrichtung des Rotors gegenüber dem Betrieb im ersten Fördermodus umgekehrt ist.

Zudem bezieht sich die Erfindung auf ein maschinenlesbares Speichermedium, das mit Anweisungen kodiert ist, die, wenn sie von einer als Datenverarbeitungseinrichtung ausgeführten Steuereinheit ausgeführt werden, bewirken, dass die Steuereinheit den Rotor zeitlich aufeinanderfolgend in einem ersten Fördermodus sowie wenigstens in einem zweiten Fördermodus betreibt, wobei in dem zweiten Fördermodus die Rotationsrichtung des Rotors gegenüber dem Betrieb im ersten Fördermodus umgekehrt ist.

Die Erfindung bezieht sich zudem auf ein Verfahren zum Betrieb einer Blutpumpe mit einem um eine Rotationsachse rotierend antreibbaren Rotor zur Blutförderung, bei dem der Rotor zeitlich aufeinanderfolgend in einem ersten Fördermodus sowie wenigstens in einem zweiten Fördermodus betrieben wird, wobei in dem zweiten Fördermodus die Rotationsrichtung des Rotors gegenüber dem Betrieb im ersten Fördermodus umgekehrt ist.

Eine besondere Ausgestaltung des Verfahrens kann dabei vorsehen, dass der Rotor regelmäßig abwechselnd im ersten und zweiten Fördermodus A, B betrieben wird, wobei insbesondere der zeitliche Anteil des Betriebs im ersten Fördermodus größer ist als der zeitliche Anteil des Betriebs im zweiten Fördermodus, insbesondere wenigstens 5-mal größer oder wenigstens 10-mal größer oder wenigstens 100-mal größer.

Die Erfindung wird nachfolgend anhand von Ausführungsbeispielen in Figuren einer Zeichnung gezeigt und detailliert erläutert.

### Dabei zeigt

- Fig. 1: einen Rotor einer Blutpumpe in einer Frontansicht schematisch,
- Fig. 2: eine in einen Patientenkörper implantierte Blutpumpe in einer Querschnittsansicht,
- Fig. 3: zwei Kennlinien einer Blutpumpe für einen Betrieb in einem ersten und einem zweiten Fördermodus sowie
- Fig. 4: fünf Zeitdiagramme, die jeweils eine Drehzahlsequenz einer Blutpumpe über eine Zeitdauer darstellen.

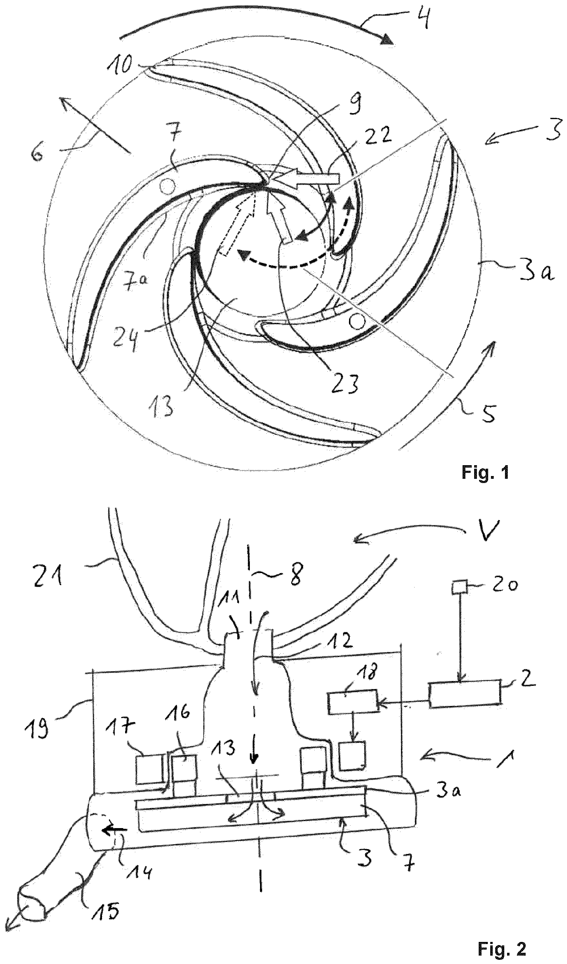

Fig. 1 zeigt den Rotor 3 einer implantierbaren VAD-Blutpumpe mit vier Förderelementen in Form von Schaufeln, von denen das Förderelement 7 beispielhaft näher betrachtet wird. Die Blickrichtung der Fig. 1 ist parallel oder entlang der Rotationsachse 8, die aus der Fig. 2 ersichtlich ist und in der Fig. 1 auf der Zeichenebene senkrecht steht. Der Rotor 3 weist in dem gezeigten Beispiel einen scheibenförmigen Träger 3a auf, der auch in Fig. 2 ersichtlich ist und auf dem mehrere Förderelemente symmetrisch angeordnet sind. In einem ersten Fördermodus A, in dem die Blutpumpe optimiert und mit bestmöglichem Wirkungsgrad betrieben wird, ist die Rotationsrichtung in Fig. 1 mit dem Pfeil 4 bezeichnet. Das Blut strömt, wie in Fig. 2 ersichtlich ist, aus einem Ventrikel V eines Herzens durch eine Ansaugöffnung 11 der Blutpumpe 1 in Richtung des Pfeils 12 ein. Weiter strömt das Blut durch die Durchströmöffnung 13 des Rotors 3 und wird von dort durch die Rotation des Rotors und der Förderelemente und die damit verknüpfte Zentrifugalbeschleunigung in radialer Richtung 6 des Rotors gefördert. In Fig. 2 ist mit dem Pfeil 14 die Ausströmrichtung des Blutes zu einem Pumpenauslass 15 bezeichnet.

Der Rotor 3 wird mittels eines Ankers 16 bewegt, der seinerseits durch einen Stator 17 angetrieben wird, der außerhalb des Fluidkanals angeordnet ist. Die Blutpumpe 1 ist mitsamt der Antriebssteuerung 18 in einem schematisch dargestellten Pumpengehäuse 19 angeordnet, wobei das Pumpengehäuse 19 derart gestaltet ist, dass es körperverträglich in den Körper eines Patienten implantiert werden kann. Dargestellt ist eine Anordnung des Antriebs auf der Einlassseite der Pumpe, vom Rotor gesehen, wobei jedoch auch eine Anordnung auf der Auslassseite der Pumpe, vom Rotor aus gesehen, denkbar ist.

Die Blutpumpe wird durch eine Steuereinheit 2 angesteuert, die sowohl innerhalb des Pumpengehäuses 19, als auch außerhalb des Pumpengehäuses im Körper des Patienten oder auch außerhalb des Körpers angeordnet sein kann.

Die Steuereinheit 2 kann zudem mit einem Sensor 20 verbunden sein, der die Kontraktionsbewegung des Herzens 21 des Patienten detektiert, so dass der Betrieb der Blutpumpe 1 mit der Kontraktionsbewegung des Herzens 21 synchronisiert werden kann. Dabei ist die Bewegungsumkehr des Rotors während der Systole denkbar, jedoch auch eine Bewegungsumkehr während der Diastole, die ein stärkeres Ausspülen der Pumpe zur Folge hat.

In Fig. 1 ist detailliert dargestellt, wie die Anströmkante oder der Anströmbereich des Förderelementes 7 im Bereich des Punktes 9 radial innen bezüglich des Rotors 3 durch das in die Pumpe einströmende Blut angeströmt wird. Je nach der Rotationsgeschwindigkeit des Rotors stellt sich bei einer Rotation des Rotors in der durch den Pfeil 4 gezeigten Richtung, das heißt im ersten Fördermodus A, die Hauptanströmrichtung des Blutes relativ zum Förderelement 7 zwischen den Pfeilen 22 und 23 ein. Ein bestimmter Bereich des Förderelementes 7 auf seiner Seite 7a, die im ersten Fördermodus die Saugseite der Schaufel 7 bildet, wird beim Betrieb in der Rotationsrichtung 4 auch bei variierender Drehzahl nicht erreicht. Wird dagegen die Rotationsrichtung zu negativen Drehzahlen umgekehrt, wie durch den Pfeil 5 angedeutet, so wird ein wesentlich erweiterter Bereich auf der Seite 7a des Förderelementes 7 angeströmt, die in diesem Betrieb die Druckseite bildet. Der erweiterte Anströmbereich ist durch den Pfeil 24 bezeichnet. Wird das gesamte Spektrum von zwei einander entgegengesetzten Rotationsrichtungen des Rotors genutzt, so kann der Winkelbereich zwischen den Pfeilen 22 und 24 ausgenutzt und das Förderelement 7 aus allen zwischen den Pfeilen 22 und 24 liegenden Richtungen angeströmt werden. Damit ergeben sich erheblich verbesserte Möglichkeiten, an dem Rotor Totwassergebiete zu vermeiden.

Es ist festzustellen, dass in beiden Rotationsrichtung 4, 5 des Rotors 3 die Förderrichtung des Rotors jeweils radial von innen nach außen, wie durch den Pfeil 6 dargestellt, beibehalten wird. Die Effizienz und Leistungsfähigkeit der Pumpe ist bei der gezeigten Gestalt der Förderelemente 7 jedoch in den Fördermodi A und B unterschiedlich. Die Rotationsrichtung 4 des Rotors 3 entspricht dem ersten Fördermodus A und damit dem Betrieb, der überwiegend eingestellt wird, während die Rotationsrichtung 5 dem zweiten Fördermodus B und damit dem weniger effizienten Fördermodus entspricht.

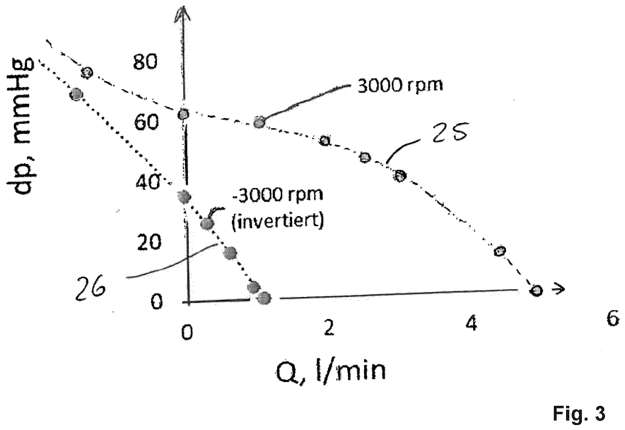

Fig. 3 zeigt Kennlinien der dargestellten Pumpen für die beiden verschiedenen Rotationsrichtungen 3 und 4, wobei die obere Linie 25 der Rotationsrichtung 4 aus der Figur 3 und dem ersten Fördermodus A entspricht, während die untere Linie 26 der Rotationsrichtung 5 und dem zweiten Fördermodus B entspricht. In Fig. 3 sind auf der horizontalen Achse die Förderrate der Pumpe und auf der vertikalen Achse der erzeugte Druck aufgetragen. Der Arbeitspunkt bei einer Rotationsgeschwindigkeit von 3000 U/min. ist in beiden Linien markiert und es ist ersichtlich, dass die Druckerzeugung und auch die Förderleistung im Fall der Kurve 26 gegenüber der Kurve 25 deutlich reduziert ist.

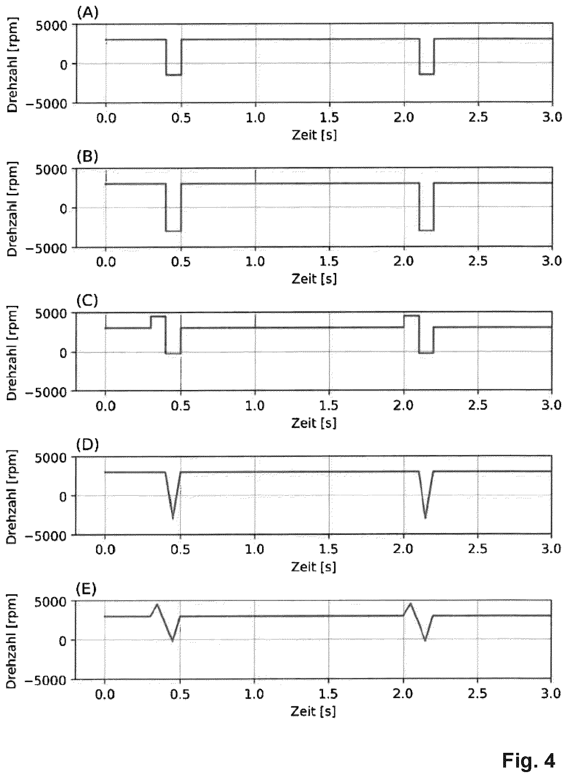

In Fig. 4 sind übereinander fünf Diagramme dargestellt, in denen jeweils der Verlauf der Drehzahl des Rotors über eine Zeit von 2,5 bis 3 Sekunden dargestellt ist. Im Fall des obersten Diagramms entspricht die obere Linie dem ersten Fördermodus A, wobei über den größten Teil der Zeit eine Drehzahl von etwas mehr als 2500 U/min. eingestellt ist. Nach etwa 0,4 Sekunden wird die Drehzahl reduziert und umgekehrt bis auf etwa 2000 U/min. in der umgekehrten Drehrichtung. Nach weiteren etwa 0,1 Sekunden wird die Drehzahl wieder umgekehrt und die Pumpe weiter im ersten Fördermodus A betrieben. Dieses Schema wird etwa alle 1,5 Sekunden wiederholt.

Im zweiten Diagramm ist dargestellt, dass anfangs die Pumpe im ersten Fördermodus A mit etwas mehr als 2500 Umdrehungen pro Minute betrieben wird, dass nach etwa 0,4 Sekunden die Drehzahl reduziert und auf etwa 3000 U/min. in der entgegengesetzten Richtung gebracht und dabei umgekehrt wird. Die Pumpe befindet sich dann etwa von 0,4 Sekunden bis 0,5 Sekunden im zweiten Fördermodus B und wird danach abgebremst und in der gegenläufigen Richtung betrieben, so dass wieder ein Übergang zum Fördermodus A stattfindet, der zeitlich überwiegend den Betrieb der Pumpe charakterisiert. Dieses Schema wird ebenfalls etwa alle 1,5 Sekunden wiederholt.

Im dritten Diagramm ist ein Schema dargestellt, bei dem zunächst die Pumpe im ersten Fördermodus A bei einer Drehzahl von etwa 3000 U/min. betrieben wird, um dann kurz vor der Umkehr der Rotationsrichtung die Drehzahl auf etwa 4000 U/min. zu erhöhen und diese erhöhte Drehzahl nach Art eines Pulses für etwa eine Zehntelsekunde beizubehalten. Danach wird die Drehzahl gesenkt und umgekehrt, wobei die umgekehrte Drehzahl betragsmäßig auf einige Umdrehungen pro Minute, beispielsweise weniger als 500U/min. oder weniger als 300 U/min. begrenzt bleibt. Dieser Fördermodus B mit umgekehrter Rotationsrichtung wird ebenfalls etwa eine Zehntelsekunde beibehalten und zum Zeitpunkt 0,5 Sekunden erfolgt dann ein Übergang zum Fördermodus A, der den größten Teil der Zeit beibehalten wird. Dieses Schema wird ebenfalls etwa alle 1,5 Sekunden wiederholt.

Es ist festzustellen, dass die Wiederholungsfrequenz auch höher sein kann, als einmal pro 1,5 Sekunden. Andererseits kann die Umkehrung der Rotationsrichtung auch deutlich seltener erfolgen, beispielsweise kann zwischen den Zeitintervallen, in denen die Rotationsrichtung umgekehrt ist, auch mindestens eine halbe Minute oder wenigstens eine Minute oder sogar ein Intervall von wenigstens mehreren Minuten oder wenigstens einer Stunde liegen. Dies hängt im Wesentlichen von der Gestaltung des Pumpenrotors und der Neigung zu Thrombenbildung ab.

Im vierten Diagramm ist dargestellt, dass, ausgehend von einem Betrieb im ersten Fördermodus bei einer Drehzahl von etwa 3000 U/min über eine steile Rampe die Drehzahl auf Null abgebremst und auf negative Drehzahlen/in der Umkehrrichtung bis auf eine betragsmäßige Drehzahl von wenigsten 1000 U/min, im gezeigten Beispiel bis auf etwa 3500 U/min beschleunigt wird, um bei Erreichen einer maximalen Drehzahl in Umkehrrichtung unmittelbar wieder abgebremst und über eine weitere Rampe auf die Ausgangsdrehzahl im ersten Fördermodus beschleunigt zu werden. Im zweiten Fördermodus B bei umgekehrter Rotationsrichtung ist somit kein Zeitintervall mit konstanter Drehzahl vorgesehen.

Ein strukturell ähnlicher Verlauf wie im vierten Diagramm ist auch im fünften Diagramm dargestellt, wobei im ersten Fördermodus vor dem Abbremsen zunächst eine pulsartige kurze Erhöhung der Drehzahl stattfindet und der Rotor danach über eine Rampe unter Umkehrung der Rotationsrichtung auf negative Drehzahlen gebracht wird. In der umgekehrten Rotationsrichtung ist dabei der Betrag der Drehzahl auf wenige 100 U/min, beispielsweise 500 U/min oder 300 U/min begrenzt. Die betragsmäßig höchste Drehzahl in Umkehrrichtung wird auch in diesem Beispiel nur punktuell erreicht, ohne dass sich eine konstante Drehzahl über ein Zeitintervall einstellt. Damit können sich während der Rotationsumkehr keine stationären Strömungsbedingungen einstellen, was der Bildung von Totwassergebieten weiter entgegenwirkt.

Durch das beschriebene System und das Verfahren zur Ansteuerung des Pumpenrotors einer Blutpumpe kann die Thrombenbildung verhindert oder wesentlich reduziert werden. Wie oben gezeigt wurde, können durch die Umkehrung der Rotationsrichtung am RotorTotwassergebiete in Bereichen vermieden werden, die bei gleichbleibender Rotationsrichtung der Pumpe nicht erreicht werden können.

## Patentansprüche

1. System umfassend eine Blutpumpe (1) mit einem um eine Rotationsachse (8) rotierend antreibbaren Rotor (3) zur Blutförderung sowie eine Steuereinheit (2) für eine Blutpumpe, wobei die Steuereinheit dazu eingerichtet ist, den Rotor zeitlich aufeinanderfolgend, insbesondere mehrfach abwechselnd, in einem ersten Fördermodus (A) sowie wenigstens in einem zweiten Fördermodus (B) zu betreiben, **dadurch gekennzeichnet, dass** in dem zweiten Fördermodus die Rotationsrichtung (4, 5) des Rotors gegenüber dem Betrieb im ersten Fördermodus umgekehrt ist.

2. System nach Anspruch 1, **dadurch gekennzeichnet, dass** der Rotor derart eingerichtet ist, dass die Förderrichtung der Blutpumpe (1) im zweiten Fördermodus (B) dieselbe ist wie im ersten Fördermodus (A).

3. System nach Anspruch 1 oder 2, **dadurch gekennzeichnet, dass** der Rotor (3) zur Förderung einer Flüssigkeit, insbesondere von Blut entweder in radialer Richtung (6) oder in axialer Richtung oder in diagonaler Richtung eingerichtet ist.

4. System nach einem der Ansprüche 1 bis 3, **dadurch gekennzeichnet, dass** der Rotor (3) ein oder mehrere Förderelemente (7), insbesondere in Form von Schaufeln, aufweist.

5. System nach Anspruch 4, **dadurch gekennzeichnet, dass** wenigstens eine, insbesondere zwei oder mehr als zwei Schaufeln (7) des Rotors oder die im ersten Fördermodus (A) auf der Druckseite der jeweiligen Schaufeln liegenden Förderflächen sich jeweils, in Blickrichtung parallel zur Rotationsachse (8) gesehen, von einem ersten Punkt (9), der einen ersten Abstand zur Rotationsachse (8) aufweist, in geradem oder gekrümmtem Verlauf bis zu einem zweiten Punkt (10) erstrecken, der einen zweiten, größeren Abstand zur Rotationsachse aufweist als der erste Punkt.

6. System nach Anspruch 5, **dadurch gekennzeichnet, dass** die Schaufeln (7) des Rotors oder die im ersten Fördermodus auf der Druckseite der jeweiligen Schaufeln liegenden Förderflächen sich jeweils, in Blickrichtung parallel zur Rotationsachse (8) gesehen, von der Rotationsachse aus radial nach außen erstrecken und insbesondere zumindest abschnittsweise in ihrem Verlauf eine bezüglich des Rotors tangentiale Komponente aufweisen.

7. Steuereinheit (2) für eine Blutpumpe (1) mit einem um eine Rotationsachse (8) rotierend antreibbaren Rotor (3) zur Blutförderung, **dadurch gekennzeichnet, dass** sie dazu eingerichtet ist, den Rotor zeitlich aufeinanderfolgend in einem ersten Fördermodus (A) sowie wenigstens in einem zweiten Fördermodus (B) zu betreiben, **dadurch gekennzeichnet, dass** in dem zweiten Fördermodus die Rotationsrichtung (4,5) des Rotors gegenüber dem Betrieb im ersten Fördermodus umgekehrt ist.

8. Maschinenlesbares Speichermedium, das mit Anweisungen kodiert ist, die, wenn sie von einer als Datenverarbeitungseinrichtung ausgeführten Steuereinheit (2) ausgeführt werden, bewirken, dass die Steuereinheit den Rotor (3) zeitlich aufeinanderfolgend in einem ersten Fördermodus (A) sowie wenigstens in einem zweiten Fördermodus (B) betreibt, **dadurch gekennzeichnet, dass** in dem zweiten Fördermodus die Rotationsrichtung (4, 5) des Rotors gegenüber dem Betrieb im ersten Fördermodus umgekehrt ist.

9. Verfahren zum Betrieb einer Blutpumpe (1) mit einem um eine Rotationsachse (8) rotierend antreibbaren Rotor (3) zur Blutförderung, bei dem der Rotor zeitlich aufeinanderfolgend in einem ersten Fördermodus (A) sowie wenigstens in einem zweiten Fördermodus (B) betrieben wird, **dadurch gekennzeichnet, dass** in dem zweiten Fördermodus die Rotationsrichtung (4, 5) des Rotors gegenüber dem Betrieb im ersten Fördermodus umgekehrt ist.

10. Verfahren nach Anspruch 9, **dadurch gekennzeichnet, dass** der Rotor (3) regelmäßig abwechselnd im ersten und zweiten Fördermodus (A, B) betrieben wird, wobei insbesondere der zeitliche Anteil des Betriebs im ersten Fördermodus größer ist als der zeitliche Anteil des Betriebs im zweiten Fördermodus, insbesondere wenigstens 5-mal größer oder wenigstens 10-mal größer oder wenigstens 100-mal größer.
